# EUROPEAN PATENT SPECIFICATION

(11) **EP 0 832 267 B1**
(45) Date of publication and mention of the grant of the patent: **06.02.2002**
(21) Application number: 96915934.2
(22) Date of filing: 07.06.1996
(51) Int. Cl.: C12N 15/86

(54) **ADENOVIRUS VECTORS FOR GENE THERAPY**
ADENOVIRALE VEKTOREN FÜR DIE GENTHERAPIE
VECTEURS ADENOVIRAUX POUR THERAPIE GENIQUE

(30) Priority: 07.06.1995 US 473168
(43) Date of publication of application: 01.04.1998
(73) Proprietor: Graham, Frank L., Hamilton, Ontario L8P 2V4 (CA); Anton, Martina, 81657 München (DE); Rudnicki, Michael A., Ottawa, Ontario K1H 8L6 (CA)
(72) Inventor: Graham, Frank L., Hamilton, Ontario L8P 2V4 (CA); Anton, Martina, 81657 München (DE); Rudnicki, Michael A., Ottawa, Ontario K1H 8L6 (CA)
(74) Representative: Baldock, Sharon Claire
(86) International application number: PCT/CA96/00375
(87) International publication number: WO 96/40955

(56) References cited:
- EP-A- 0 704 534
- WO-A-95/00655
- WO-A-95/27071
- PROC. NATL.ACAD SCI., vol. 91, September 1994, NATL. ACAD SCI.,WASHINGTON,DC,US;, pages 8802-8806, XP002011773 A.J. BETT ET AL.: "An efficient and flexible system for construction of adenovirus vectors with insertions or deletions in early regions 1 and 3" cited in the application
- NUCLEIC ACIDS RESEARCH, vol. 23, no. 19, 11 October 1995, IRL PRESS LIMITED,OXFORD,ENGLAND, pages 3816-3821, XP002011774 Y. KANEGAE ET AL.: "Efficient gene activation in mammalian cells by using recombinant adenovirus expressing site-specific Cre recombinase"
- J. VIROLOGY, vol. 69, no. 8, August 1995, AM.SOC.MICROBIOL.,WASHINGTON,US, pages 4600-4606, XP002011775 M. ANTON AND F.L. GRAHAM: "Site-specific recombination mediated by an adenovirus vector expressing the Cre recombinase protein: a molecular switch for control of gene expression" cited in the application

## Description

### FIELD OF THE INVENTION

The present invention relates to adenovirus vectors that have increased utility for gene transfer into mammalian cells. The vector systems described have increased capacity for insertion of foreign DNA and improved safety.

### BACKGROUND OF THE INVENTION

Parent Application Serial No. 08/250,885, filed on May 31, 1994 and published as WO 95/00665, discloses and claims a genus of adenovirus (Ad)-derived cell expression vectors having excellent potential as live recombinant vaccines and as transducing vectors for gene therapy. In the human Ad genome, early region 1 (E1), E3, and a site upstream of E4 have been utilized as sites for introducing foreign DNA sequences to generate adenovirus recombinants. In the absence of compensating deletions in E1 or E3 a maximum of about 2 kb can be inserted into the Ad genome to generate viable virus progeny. The E1 region is not required for viral replication in complementing 293 cells and up to 3.2 kb can be deleted in this region to generate conditional helper independent vectors with a capacity of 5.0-5.2 kb. In the E3 region, which is not required for viral replication in cultured cells, deletions of various sizes have been utilized to generate nonconditional helper independent vectors with a capacity of up to 4.5-4.7 kb.

The maximum capacity for inserts of foreign DNA in currently available helper independent Ad vectors such as those described in the parent application is approximately 8 kb. This limited capacity arises from the use of Ad vectors which have deletions of E1 and E3 sequences and from the fact that most other regions of the viral genome must be retained in order that the viral vector may be propagated without the need for a helper virus.

Besides this limited capacity for insert DNA, previous vectors retain most of the viral genome, expression of viral genes in transduced cells or in inoculated animals, including humans, can result in toxic or other untoward effects. In addition, previous viral vectors can recombine with Ad sequences present in cells used for propagation of the vectors or with Ad sequences that may be present in inoculated animals. Therefore, it is the objective of this invention to provide Ad cloning vectors from which all or most viral genes have been removed and which will have increased safety and capacity for larger insertions compared to currently available vectors.

### SUMMARY OF THE INVENTION

It is the goal of this invention to provide a simple and useful system by which high capacity Ad5 cloning vectors may be developed. Provision of Cre recombinase in Ad infected cells can catalyze excision or rearrangement of viral DNA sequences that contain the target sites (*loxP*) for Cre mediated site specific recombination. In the present invention, use is made of this knowledge to construct Ad5 genomes in which the viral DNA packaging signals can be excised from the viral genome by action of a recombinase. Said excision of said packaging signal results in a viral DNA that is unable to package into virion particles. Such a viral DNA, though unable to package into virions, may encode viral functions that provide complementing functions for replication of a second, viral "vector", that lacks substantial portions of the viral genome so that in coinfected cells though both helper and vector DNAs may replicate, only the vector DNA can be packaged into virions.

One embodiment of the present invention provides a bacterial plasmid comprising a circularized modified human adenovirus type 5 (Ad5) genome that contains sequences that can be recognized and acted upon by a site specific recombinase. Said bacterial plasmid is able to generate infectious Ad5 carrying the modified sequences including the sequences that can be recognized by the recombinase. The structure of the modified sequences in the bacterial plasmid and in viruses generated from said plasmid is such that recombination catalyzed by the recombinase will result in excision of sequences, known as the packaging signal, near the left end of the Ad5 genome, that are required for packaging of Ad5 DNA into infectious virion particles. Optionally, certain regions of the plasmid and resulting viruses may be deleted, such as sequences from E1 or E3 that can be omitted from the viral genome without preventing the viral genome from replicating in such cells as may be permissive for replication of said viral genome in the form of infectious virus.

A second embodiment of the invention provides a bacterial plasmid comprising approximately 340 base pairs from the left end of the Ad5 genome, including the left end terminal repeat sequences of said genome and the packaging signal sequences thereof and the right terminal repeat sequences of the Ad5 genome. The left end of the left terminal repeat sequence is joined in "head to tail" configuration with the right end of the right terminal repeat. Between approximately nucleotide 340 near the left end of the genome and approximately nucleotide 35,800 near the right end of the genome, are substituted restriction enzyme sites suitable for insertion of foreign DNA sequences.

A third embodiment of the invention provides a mammalian cell line, such as a human cell line, that provides the Cre recombinase enzyme. Alternatively, Cre may be provided by an Ad5 derived vector that expresses the Cre protein in suitable cells.

Other embodiments of the present invention include Ad genome constructs, known as "vectors", containing substantial deletions of viral DNA sequences that are substituted with large insertions of foreign DNA 20-35kb in length. Such genomes are unable to replicate as viruses in the absence of viral products provided by a second virus, hereafter called a "helper" virus.

One specific embodiment of the invention is a helper virus that can be designed, propagated, and used in such a way that when employed to support replication of a second virus, the vector, from which substantial portions of the viral genome have been deleted and substituted with foreign DNA, said "helper' virus DNA is unable to be packaged into infectious virions.

Preferred embodiments of the invention use the recombinase Cre and the target sites (*loxP*) for Cre mediated site specific recombination. It will, however, be appreciated by persons skilled in the art that other enzymes capable of catalyzing site specific recombination between DNA target sequences recognized by the said enzymes could equally be employed in place of the Cre recombinase.

### BRIEF DESCRIPTION OF FIGURES

**Fig. 1** is a diagrammatic representation of Cre mediated excision of DNA from a viral vector in which the packaging signal is flanked by *lox P* sites.

**Fig. 2** is a diagrammatic representation of a method to generate helper dependent viral vectors using Cre mediated excision of the packaging signal to prevent packaging of the helper virus DNA.

**Fig. 3** is a diagrammatic representation of a plasmid derived from pBHG10 into which *lox P* sequences have been introduced at positions flanking the packaging signal.

**Fig. 4** is a diagrammatic representation of a plasmid derived from pBHG10 from which most of the viral DNA has been deleted save for the left and right ITRs and the packaging signal.

**Fig. 5** is a diagrammatic representation of a means to obtain coreplicating helper and helper dependent viruses by cotransfection of 293Cre cells.

**Fig. 6** is a diagrammatic representation of the construction of pLC2 and pLC3 plasmids.

**Fig. 7** is a diagrammatic summary of the transformation, selection and screening protocol for Cre expressing 293 and 293N3S cell lines.

**Fig. 8** is a Western blot analysis of G418 resistant clones screened for expression of Cre with Cre specific rabbit antiserum.

**Fig. 9** is a diagrammatic representation of luciferase expression assays on parental cell lines.

**Fig. 10A** is a photomicrograph showing the results of luciferase expression assays on transformed cell lines.

**Fig. 10B** is a diagrammatic representation of luciferase expression assays on transformed cell lines.

**Fig. 11A** is a diagrammatic representation of the construction of AdLC8 helper virus.

**Fig. 11B** is a photomicrograph showing the ethidium bromide staining results demonstrating the efficiency of excision of packaging signals from AdLC8.

**Fig. 11C** is a photomicrograph showing Southern blot analysis results demonstrating the kinetics of Cre-mediated recombination.

**Fig. 12A** is a diagrammatic representation of the pRP1001 transfection of 293Cre1 cells and of the AdLC3 infection of the transfected cells 18 hours later.

**Fig. 12B** is a graph showing the titer increase resulting from the serial passage of AdRP1001 through AdLC8-infected 293 Cre1 cells.

**Fig. 13A** is a diagrammatic representation of the amplification of the AdRP1001 vector to high titers with AdLC8cluc.

**Fig. 13B** is a graph showing the titer increase resulting from the serial passage of AdRP1001 through AdLC8cluc-infected 234Cre1 cells.

**Fig. 14** is a graph showing the relationship between luciferase activity and contaminating helper virus.

### DETAILED DESCRIPTION OF THE INVENTION

It is important to an understanding of the present invention to note that all technical and scientific terms used herein, unless otherwise defined, are intended to have the same meaning as commonly understood by one of ordinary skill in the art. The techniques employed herein are also those that are known to one of ordinary skill in the art, unless stated otherwise.

Reference to particular buffers, media, reagents, cells, culture conditions and the like, or to some subclass of same, is not intended to be limiting, but should be read to include all such related materials that one of ordinary skill in the art would recognize as being of interest or value in the particular context in which that discussion is presented. For example, it is often possible to substitute one buffer system or culture medium for another, such that a different but known way is used to achieve the same goals as those to which the use of a suggested method, material or composition is directed.

The terms used herein are not intended to be limiting of the invention. For example, the term "gene" includes cDNAs, RNA, or other polynucleotides that encode gene products. "Foreign gene" denotes a gene that has been obtained from an organism or cell type other than the organism or cell type in which it is expressed; it also refers to a gene from the same organism that has been translocated from its normal situs in the genome. In using the terms "nucleic acid", "RNA", "DNA", *etc*., we do not mean to limit the chemical structures that can be used in particular steps. For example, it is well known to those skilled in the art that RNA can generally be substituted for DNA, and as such, the use of the term "DNA" should be read to include this substitution. In addition, it is known that a variety of nucleic acid analogues and derivatives can be made and will hybridize to one another and to DNA and RNA, and the use of such analogues and derivatives is also within the scope of the present invention. "Expression" of a gene or nucleic acid encompasses not only cellular gene expression, but also the transcription and translation of nucleic acid(s) in cloning systems and in any other context. The term "recombinase" encompasses enzymes that induce, mediate or facilitate recombination, and other nucleic acid modifying enzymes that cause, mediate or facilitate the rearrangement of a nucleic acid sequence, or the excision or insertion of a first nucleic acid sequence from or into a second nucleic acid sequence. The "target site" of a recombinase is the nucleic acid sequence or region that is recognized (e.g., specifically binds to) and/or acted upon (excised, cut or induced to recombine) by the recombinase. The term "gene product" refers primarily to proteins and polypeptides encoded by a nucleic acid, but further encompasses nucleic acids encoded by other nucleic acids (e.g., non-coding and regulatory RNAs such as tRNA, sNRPs). The term "regulation of expression" refers to events or molecules that increase or decrease the synthesis, degradation, availability or activity of a given gene product.

The present invention is also not limited to the use of the cell types and cell lines used herein. Cells from different tissues (breast epithelium, colon, lymphocytes, *etc*.) or different species (human, mouse, *etc*.) are also useful in the present invention.

It is important in this invention to detect the generation and expression of recombinant nucleic acids and their encoded gene products. The detection methods used herein include, for example, cloning and sequencing, ligation of oligonucleotides, use of the polymerase chain reaction and variations thereof (*e*.*g*., a PCR that uses 7-deaza GTP), use of single nucleotide primer-guided extension assays, hybridization techniques using target-specific oligonucleotides that can be shown to preferentially bind to complementary sequences under given stringency conditions, and sandwich hybridization methods.

Sequencing may be carried out with commercially available automated sequencers utilizing labelled primers or terminators, or using sequencing gel-based methods. Sequence analysis is also carried out by methods based on ligation of oligonucleotide sequences which anneal immediately adjacent to each other on a target DNA or RNA molecule (Wu and Wallace, Genomics 4: 560-569 (1989); Landren et al., Science 241: 1077-1080 (1988); Nickerson et al., Proc. Natl. Acad. Sci. 87: 8923-8927 (1990); Barany, F., Proc. Natl. Acad. Sci. 88: 189-193 (1991)). Ligase-mediated covalent attachment occurs only when the oligonucleotides are correctly base-paired. The Ligase Chain Reaction (LCR), which utilizes the thermostable Taq ligase for target amplification, is particularly useful for interrogating late onset diabetes mutation loci. The elevated reaction temperatures permits the ligation reaction to be conducted with high stringency (Barany, F., PCR Methods and Applications 1: 5-16 (1991)).

The hybridization reactions may be carried out in a filter-based format, in which the target nucleic acids are immobilized on nitrocellulose or nylon membranes and probed with oligonucleotide probes. Any of the known hybridization formats may be used, including Southern blots, slot blots, "reverse" dot blots, solution hybridization, solid support based sandwich hybridization, bead-based, silicon chip-based and microtiter well-based hybridization formats.

The detection oligonucleotide probes range in size between 10-1,000 bases. In order to obtain the required target discrimination using the detection oligonucleotide probes, the hybridization reactions are generally run between 20°-60° C, and most preferably between 30°-50° C. As known to those skilled in the art, optimal discrimination between perfect and mismatched duplexes is obtained by manipulating the temperature and/or salt concentrations or inclusion of formamide in the stringency washes.

The cloning and expression vectors described herein are introduced into cells or tissues by any one of a variety of known methods within the art. Such methods are described for example in Sambrook et al., Molecular Cloning: A Laboratory Manual, Cold Spring Harbor Laboratory, New York (1992), which is hereby incorporated by references, and in Ausubel et al., Current Protocols in Molecular Biology, John Wiley and Sons, Baltimore, MD (1989), which is also hereby incorporated by reference. The methods include, for example, stable or transient transfection, lipofection, electroporation and infection with recombinant viral vectors.

The protein products of recombined and unrecombined coding sequences may be analyzed using immune techniques. For example, a protein, or a fragment thereof is injected into a host animal along with an adjuvant so as to generate an immune response. Immunoglobulins which bind the recombinant fragment are harvested as an antiserum, and are optionally further purified by affinity chromatography or other means. Additionally, spleen cells may be harvested from an immunized mouse host and fused to myeloma cells to produce a bank of antibody-secreting hybridoma cells. The bank of hybridomas is screened for clones that secrete immunoglobulins which bind the recombinantly produced fragment. More specifically, immunoglobulins that selectively bind to the variant polypeptides but poorly or not at all to wild-type polypeptides are selected, either by pre-absorption with wild-type proteins or by screening of hybridoma cell lines for specific idiotypes that bind the variant, but not wild-type, polypeptides.

Nucleic acid sequences capable of ultimately expressing the desired variant polypeptides are formed from a variety of different polynucleotides (genomic or cDNA, RNA, synthetic oligonucleotides, *etc*.) as well as by a variety of different techniques.

The DNA sequences are expressed in hosts after the sequences have been operably linked to (*i.e.*, positioned to ensure the functioning of) an expression control sequence. These expression vectors are typically replicable in the host organisms either as episomes or as an integral part of the host chromosomal DNA. Commonly, expression vectors contain selection markers (*e.g.*, markers based on tetracycline resistance or hygromycin resistance) to permit detection and/or selection of those cells transformed with the desired DNA sequences. Further details can be found in U.S. Patent No. 4,704,362.

Polynucleotides encoding a variant polypeptide include sequences that facilitate transcription (expression sequences) and translation of the coding sequences such that the encoded polypeptide product is produced. Construction of such polynucleotides is well known in the art. For example, such polynucleotides include a promoter, a transcription termination site (polyadenylation site in eukaryotic expression hosts), a ribosome binding site, and, optionally, an enhancer for use in eukaryotic expression hosts, and, optionally, sequences necessary for replication of a vector.

*E. coli* is one prokaryotic host useful particularly for cloning DNA sequences of the present invention. Other microbial hosts suitable for use include bacilli, such as *Bacillus subtilus,* and other enterobacteriaceae, such as *Salmonella*, *Serratia,* and various *Pseudomonas* species. Expression vectors are made in these prokaryotic hosts which will typically contain expression control sequences compatible with the host cell (*e.g.*, an origin of replication). In addition, any number of a variety of well-known promoters are used, such as the lactose promoter system, a tryptophan (Trp) promoter system, a beta-lactamase promoter system, or a promoter system from phage lambda. The promoters typically control expression, optionally with an operator sequence, and have ribosome binding site sequences, for example, for initiating and completing transcription and translation.

Other microbes, such as yeast, are used for expression. *Saccharomyces* is a suitable host, with suitable vectors having expression control sequences, such as promoters, including 3-phosphoglycerate kinase or other glycolytic enzymes, and an origin of replication, termination sequences, *etc.* as desired.

In addition to microorganisms, mammalian tissue cell culture is used to express and produce the polypeptides of the present invention. Eukaryotic cells are preferred, because a number of suitable host cell lines capable of secreting intact human proteins have been developed in the art, and include the CHO cell lines, various COS cell lines, HeLa cells, myeloma cell lines, Jurkat cells, and so forth. Expression vectors for these cells include expression control sequences, such as an origin of replication, a promoter, an enhancer, and necessary information processing sites, such as ribosome binding sites, RNA splice sites, polyadenylation sites, and transcriptional terminator sequences. Preferred expression control sequences are promoters derived from immunoglobulin genes, SV40, Adenovirus, Bovine Papilloma Virus, and so forth. The vectors containing the DNA segments of interest (*e.g.,* polypeptides encoding a variant polypeptide) are transferred into the host cell by well-known methods, which vary depending on the type of cellular host. For example, calcium chloride transfection is commonly utilized for prokaryotic cells, whereas calcium phosphate treatment or electroporation is useful for other cellular hosts.

The method lends itself readily to the formulation of test kits for use in diagnosis. Such a kit comprises a carrier compartmentalized to receive in close confinement one or more containers wherein a first container contains suitably labeled DNA probes. Other containers contain reagents useful in the localization of the labeled probes, such as enzyme substrates. Still other containers contain restriction enzymes, buffers *etc.*, together with instructions for use.

The recombinant Ad vectors described herein are significantly different from previously described constructs. They combine the use of vectors having deletions of all or most of the viral genes with helper viruses that are designed so that, when used in coinfections with vector viruses, said helper viruses are able to complement the growth of the vectors but are unable to package their viral DNA into infectious virions. Thus vector viruses can be prepared substantially free of helper virus.

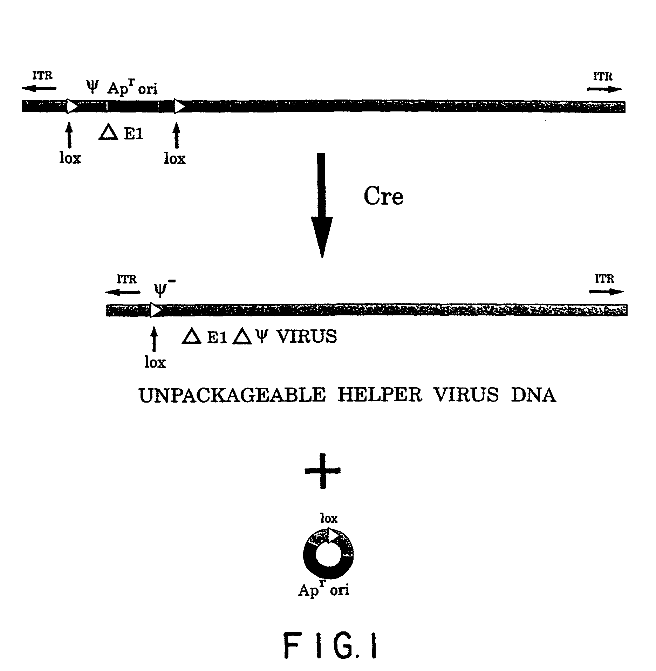

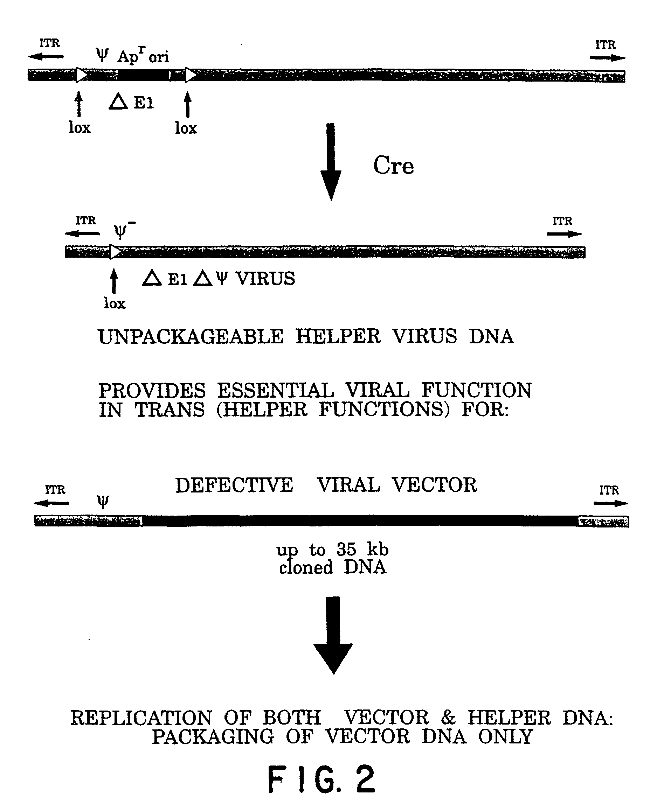

For viral DNA replication and packaging of viral DNA into virion particles, only three regions of the viral DNA are known to be required in *cis*. These are the left inverted terminal repeat , or ITR, (bp 1 to approximately 103) the packaging signals (approximately 194 to 358 bp) (Hearing and Shenk, 1983, Cell 33: 695-703; Grable and Hearing 1992, J. Virol. 64: 2047-2056) and the right ITR. All other regions of the viral genome appear to be required only to produce viral products that act in *trans* to allow viral replication and production of infectious viruses. Thus if all essential viral proteins and RNA could be provided by a helper virus, a vector could be designed and constructed that could have most of the viral DNA deleted save for those sequences mentioned above that are required in cis for viral DNA replication and packaging. A problem with helper dependent vectors has been that preparations of such vectors are invariably contaminated with helper virus and it is technically very difficult to separate the helper from the vector. In the main embodiments of the present invention the helper virus is designed to have two target sites for site-specific recombination near the left end of the genome, one inserted at approximately 189bp from the extreme left end of the viral DNA, and the second, in parallel orientation with the first target site, situated rightward of the packaging signals, ie rightward of bp 358 (diagrammed in Figure 1). This virus will be able to replicate in cells that are normally permissive for growth of Ad5. However, in cells that express the appropriate recombinase, or in the presence of a second virus that expresses the recombinase, excision of sequences between the target sites of the helper virus DNA will remove the packaging signal, and the resulting viral DNA will fail to package into infectious viral particles. Therefore, in cells coinfected with said helper and with a second virus, a vector from whose genome have been deleted all or most of the viral DNA sequences that are normally required for expression of viral products necessary in trans for viral replication, both vector and helper viral genomes will replicate but only the vector DNA that retains the packaging signal will be packaged into virions (Figure 2).

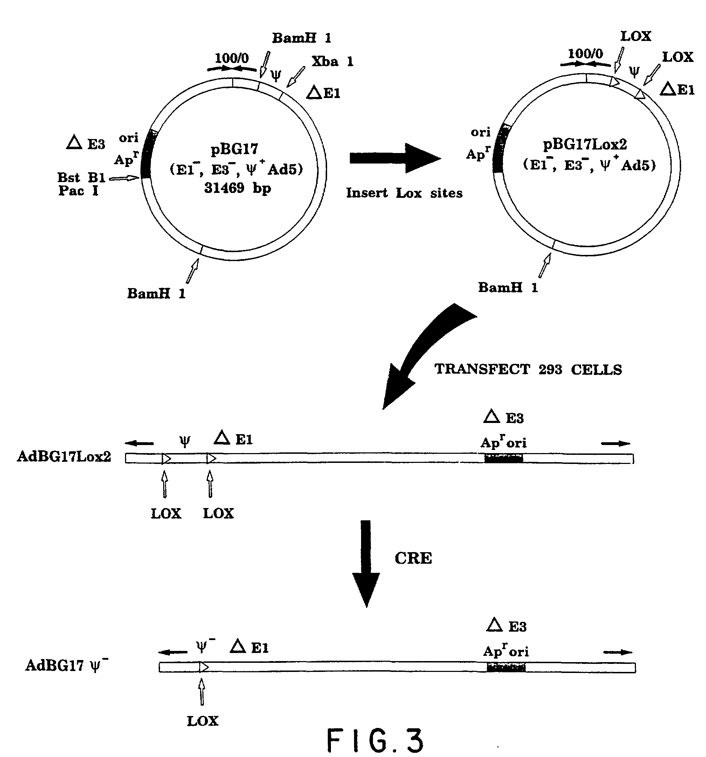

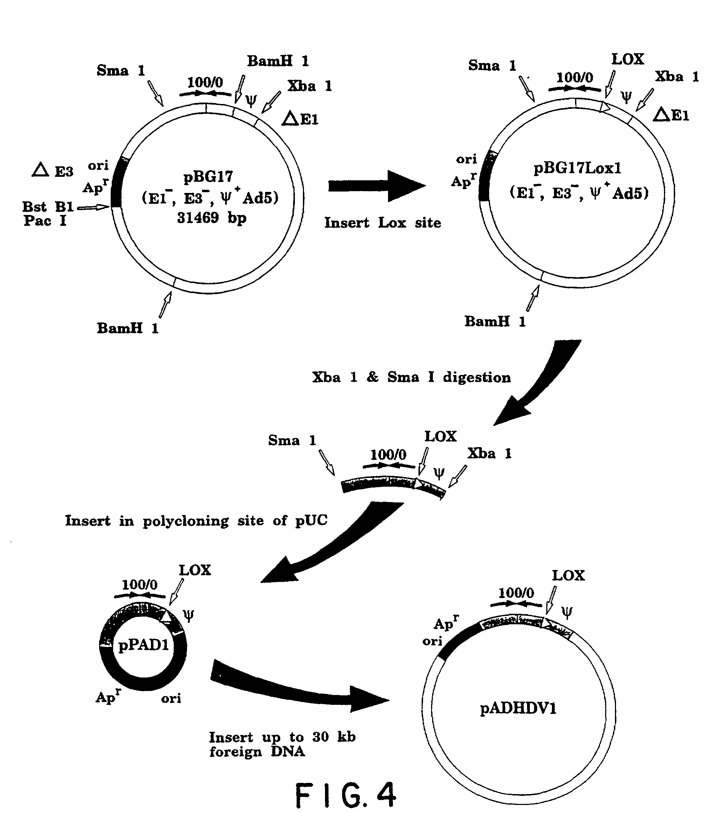

In one embodiment of the invention, the helper virus is derived from a plasmid similar to ones described in the parent application and as illustrated in Figures 3 and 4. In these examples, the Ad5 genome is present as a circular molecule containing a bacterial plasmid derived origin of DNA replication (designated "ori") and a bacterial antibiotic resistance coding sequence ("Ap^{r}") conferring to bacteria carrying said molecule resistance to ampicillin. In this example in which said circular form of the Ad5 genome is designated pBG17, viral sequences from regions E1 and E3 have been deleted from the viral genome, but this example is not meant to be limiting since other deletions or no deletions may equally be engineered in the circularized molecule by methods described in the parent application. The molecule designated pBG17 contains Ad5 sequences from bp 19 (left genomic end) to bp 341 with an artificially engineered BamH 1 restriction site inserted between approximately bp 188 and 189 in the Ad 5 sequences which is between the "ITR" and the packaging signal, "ψ", and known not to interfere with viral replication (Bett, A. J., W. Haddara, L. Prevec, and F. L. Graham. 1994, Proc. Natl. Acad. Sci. USA 91: 8802-8806). Ad 5 sequences present in pBG17 then extend rightward of the packaging signal to approximately bp 341 at which position is located an Xba 1 restriction site followed by Ad 5 sequences from approximately bp 3534 to approximately bp 27864, then sequences comprising 1874 bp of DNA containing the pUC19 origin of replication and ampicillin resistance gene, and finally Ad5 bp 30996 to 35934 (right genomic end). By techniques that are readily employed by a person skilled in the art, *lox P* sites, which are well defined DNA sequences of about 34 bp, can be introduced into the Ad5 genome at the Bam HI and XbaI sites flanking "ψ". For example synthetic double stranded oligodeoxnucleotides can be readily designed and synthesized such that they contain the *lox P* sequence recognized by cre and are flanked by single stranded extensions that allow ligation into BamHI or XbaI cleaved DNA. Thus a person skilled in the art can readily obtain the plasmid designated pBG17Lox1 (Fig. 4) having a *lox P* site introduced into the Bam HI site at nt 188, and subsequently pBG17Lox2 (Fig. 3) having an additional *lox P* site introduced into the XbaI site of pBG17Lox1. The plasmid pBG17 can be used to generate infectious virus by transfection of 293 cells. Equally, the plasmids pBG17Lox1 or pBG17Lox2, will generate infectious virus (eg. AdBG17Lox2 illustrated in Figure 3) since insertions of up to 271 bp can be engineered between the ITR and the packaging signal without interfering with viral replication and packaging of viral DNA (Hearing et al., Journal of Virology Vol 61, p 2555, 1987). In the presence of Cre enzyme the sequences containing the packaging signal will be excised as a result of intramolecular recombination between the two *lox P* sites (Fig. 3, bottom) resulting in a viral genome that retains all the sequences necessary for replication but lacks the sequences needed for packaging of DNA into virions. Said genome may serve as a complementing viral genome to support the replication of a second virus, a vector, that lacks all or most of the viral genes necessary for viral replication as diagrammed in Fig. 2. These examples are not meant to be limiting as it will be appreciated that modified viruses similar in DNA structure to that of Figure 3 can be generated by other means. For example a person skilled in the art could introduce *lox P* sites into other sites in the plasmids illustrated in Fig. 3 such as the Bst B1 or Pac I sites or into such other locations as might be desirable, or into such other plasmids containing Ad sequences, or other Ad viral genomes as might be desirable. Use of Cre recombinase in this and other examples is not meant to be limiting as a person skilled in the art will readily appreciate that other enzymes capable of catalyzing site specific recombination between DNA sequences recognized by said enzymes could equally be employed in place of the Cre recombinase. An example, not meant to be limiting, of such an enzyme that could be substituted for Cre is the "FLP" recombinase of yeast in combination with its target site (O'Gorman et al. Science 251, 1351, 1991).

Another embodiment of the invention provides human cells, such as 293 cells or other cells that may be deemed suitable in that they support the replication of the viral components of the invention, that express Cre recombinase and that can be transfected with the plasmids described in the previous examples to generate a helper virus from which the packaging signals have been removed through excision mediated by Cre. It will be appreciated by those skilled in the art that the requisite cell lines can be generated by transfecting 293 cells or other cells with a plasmid comprising the coding sequences for Cre under the control of suitable regulatory sequences including a promoter and polyadenylation signal and containing in addition a selectable gene encoding, for example, resistance to G418 or histidinol. A person skilled in the art can readily obtain drug resistant cells that will express the Cre recombinase in addition to the drug resistance gene used for selection.

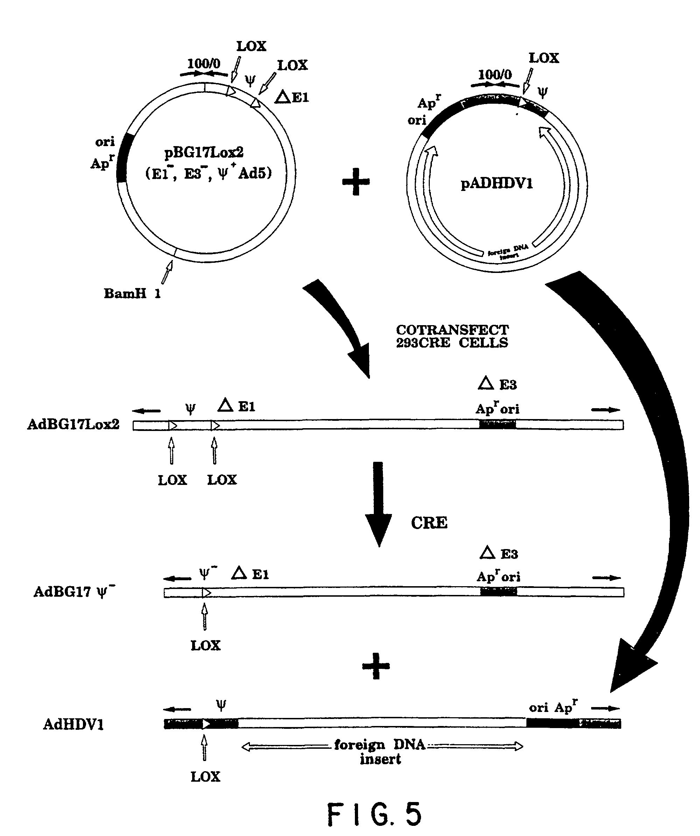

In another embodiment of the invention, a plasmid consisting of sequences comprising the left ITR, the packaging signal, and the right ITR, and optionally containing additional viral sequences can be readily obtained. An example, which is not meant to be limiting, is illustrated in Fig. 4. In this example, pBG17Loxl DNA is digested with restriction enzymes XbaI and SmaI which cleave the viral DNA in pBG17Lox1 at sites shown, as well as at other sites in viral DNA. The fragment containing the junction of viral termini (indicated by head to head arrows in Fig. 4) and the *lox P* site and packaging signal, can be purified and inserted into the polycloning site of a suitable cloning plasmid such as pUC18 or pUC19 to generate the plasmid designated as pPAD1. This example is not meant to be limiting as a person skilled in the art could equally insert said fragment into such other cloning plasmids as might be suitable or desirable. In the example illustrated, pPAD1 can serve as a vector for insertion of foreign DNA up to approximately 30 kb in size at one of the remaining restriction enzyme cloning sites present at the junctions of pUC and Ad5 DNA, to generate a plasmid such as pADHDV1, in which the open segment of pADHDV1 represents foreign DNA of arbitrary origin and sequence composition. The plasmid pADHDV1 contains all the Ad5 sequences needed in cis for viral DNA replication and packaging of viral DNA into virions. Provided that viral functions necessary in trans are supplied by a helper virus, therefore, pADHDV1 will have the potential to replicate as a helper dependent viral DNA molecule that will contain up to 30 kb of foreign DNA flanked by viral DNA sequences from the left and right ends of the viral genome. It may be advantageous to include as part of the foreign DNA inserted in pADHDV1, a DNA sequence capable of providing expression of a readily detectable reporter gene in addition to other sequences, the reporter gene providing a simple means of identifying cells or groups of cells that are infected with the virus ADHDV1 derived from pADHDV1. As an example which is not meant to be limiting, a person skilled in the art could include in pADHDV1, sequences coding for bacterial β-galactosidase, expression of which is readily detectable by exposure of cells to X-gal. Furthermore, in the example shown in Figure 4, pPAD1 and pADHDV1 contain a single *lox P* site at Ad5 nt 189, that is at the same site as for one of the *lox P* insertions in pBG17Lox1&2. Although this example is not meant to be limiting, placement of a *lox P* site at this position in pPAD1, pADHDV1, and derivatives, may serve to reduce the efficiency of recombination between helper virus and vector during coreplication of the two viruses as illustrated in Figures 2 and 5.

In another embodiment of the invention, coreplication of helper virus comprising sequences derived from a plasmid such as pBG17Lox2 and a helper dependent virus comprising sequences derived from a plasmid such as pADHDV1 may be achieved by cotransfection of cells with said plasmids to generate replicating viral genomes. In the example illustrated in Figure 5, which is not meant to be limiting, AdBG17Lox2 will, in the presence of Cre recombinase, be converted to AdBG17ψ- by excision of the sequences bracketed by *lox P* sites. The virus AdBG17ψ- will, by virtue of the removal of the packaging signals, be unable to package its genome into virions but will be able to replicate its DNA and provide viral functions necessary in trans for viral replication and thereby provide complementing functions for replication of the helper dependent virus, AdHDV1. Because AdHDV1 retains the packaging signals the DNA of this helper dependent virus will be packaged into virions. The helper dependent virus AdHDV1 may be recovered, and optionally purified and concentrated by isopycnic centrifugation in CsCl gradients to produce helper dependent virus preparations substantially free or totally free of contaminating helper virus.

In another embodiment of the invention, 293 cells or other human cells that do not express Cre may be transfected with a plasmid such as that designated as pBG17Lox2 in Figures 3 and 5 to produce a virus such as that designated as AdBG17Lox2. Said virus may replicate in said cells without undergoing excision of sequences bracketed by *lox P* and can therefore be readily propagated. Coinfection of 293Cre or equivalent cells with AdBG17Lox2 and AdHDV1 will lead to formation of AdBG17ψ- which will complement the growth of AdHDV1 resulting in coreplication of both viral genomes but packaging only of AdHDV1 DNA into viral particles.

### Example 1. Production and Characterization of Cell Lines Expressing Recombinase Cre.

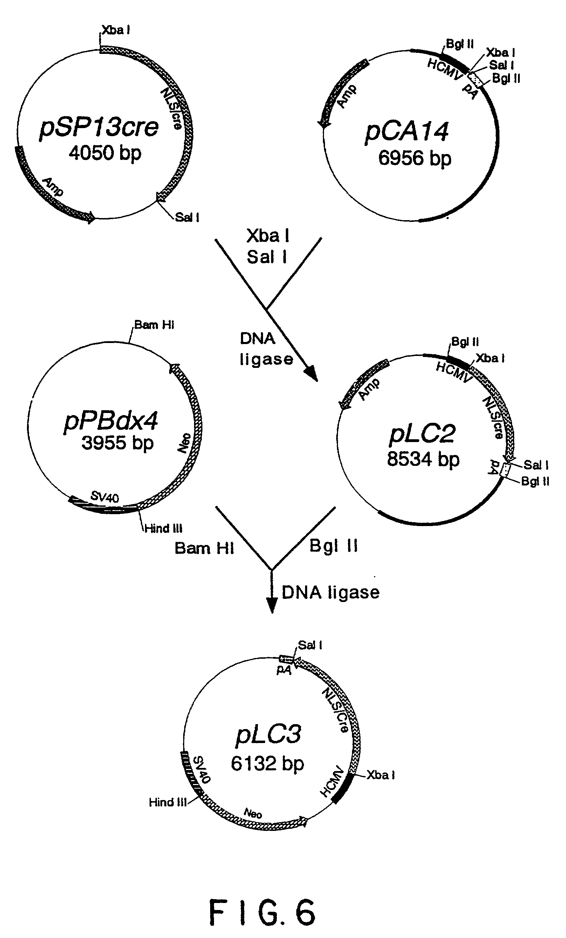

Plasmid pLC2 was created by cloning the 1.6 kb XbaI/SalI fragment of pSP13cre into the XbaI/SalI site of pCA14, placing the cre gene under the control of the HCMV promoter and adding an SV40 polyadenylation signal (pA) to the 3' end of the gene. As the XbaI/SalI segment of pSP13cre already contains an SV40 pA, this cloning step resulted in two polyadenylation signals 3' of Cre. The 2.2 kb *BglII* fragment of pLC2 was then cloned into the BamHI site of pPBdx4. The resulting plasmid pLC3, contains the Cre expression cassette plus the gene for neomycin resistance under the control of the SV40 promoter. Fig. 6 outlines the construction of the pLC2 and pLC3 plasmids. The structures of plasmids pLC2 and pLC3 were confirmed through restriction analysis.

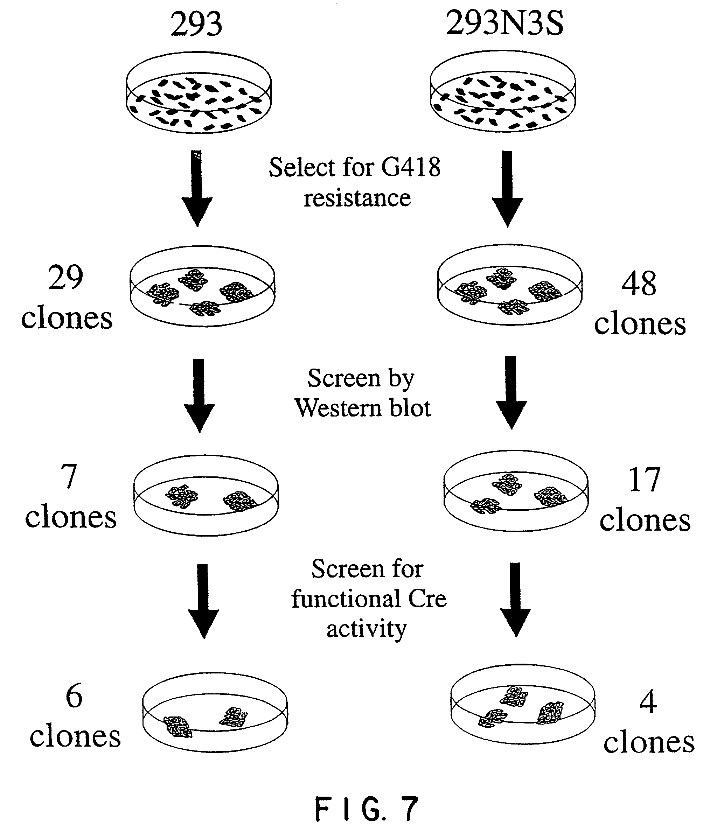

293 and 293N3S cells were maintained in F11 medium supplemented with 100 U penicillin per ml, 100 µg of streptomycin per ml, 2.5 µg/ml fungizone, and 10% fetal bovine serum for maintenance of 5% horse serum for infection. The cells were transformed with pLC2 using the calcium phosphate technique (McKinnon, R. D., and F. R. Graham, Microinjection and Organelle Transplantation Techniques 199-214 (1986)). Cells were then subjected to selection with 400 µg/ml G418 in complete F11 starting 3 days after transformation. Four weeks after transformation, G418 resistant colonies were cloned and expanded. Fig. 7 summarizes the transformation, selection and screening protocol for Cre expressing cell lines. Cell culture media and reagents with the exception of fungizone, were purchased from Gibco BRL (Burlington, Ontario, Canada). Fungizone was purchased from Bristol-Myers Squibb Canada (Montreal, Quebec, Canada).

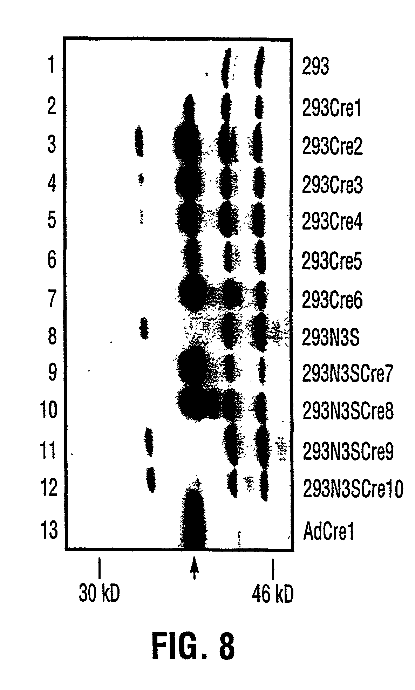

Clones were initially screened for expression of Cre by Western blot analysis with Cre specific rabbit antiserum. Of 77 G418 resistant clones isolated, 7 293 and 17 293N3S clones were found to express Cre protein of the correct size (38kD) at levels varying from line to line. Ten representative lines are shown in Fig. 8. Lines 293Cre1 to 6 (lanes 2-7) and 293N3SCre7 and 8 (lanes 9-10) were found to express high levels of Cre protein (comparable to levels in AdCre 1 infected cells; AdCre1 contains the *cre* gene under the control of the HCMV promoter in an E1 delected vector) whereas no Cre was detected in parental 293 and 293M3S cell lines (lanes 1 and 8), or in 293N3SCre9 and 10 (lanes 11-12). A small amount of Cre had been detected in the latter two cell lines when these clones were first isolated, but expression was undetectable in subsequent assays.

Cell lines that scored positive for Cre expression by Western blot were next assayed for functional recombinase activity using Ad vectors expressing reporter genes under the control of a Cre sensitive molecular switch (Anton, M. and F. L. Graham, J. Virol. 69:4600-4606 (1995)). 293Cre and 293N3SCre cell lines were infected with the vectors in combination with Add170-3 or AdCre1 at an MOI of 10 PFU per cell for each vector, to compare the levels of activity of the endogenously expressed Cre recombinase with those produced by AdCre1.

Clones that tested positive for Cre expression by Western blot analysis were then screened for Cre recombinase activity using the Cre inducible viruses AdMA19, AdMA23, and AdMA35. 293Cre and 293N3SCre cell lines were infected with the vectors in combination with Add170-3 or AdCre1 at an MOI of 10 PFU per cell for each vector, to compare the levels of activity of the endogenously expressed Cre recombinase with those produced by AdCre1. A preliminary screening using AdMA23, identified 6 293 Cre cell lines and 4 293N3SCre lines that were able mediate specific *Cre-lox* recombination and induce expression of β-galactosidase activity from AdMA23 in the absence of any extraneous source of Cre recombinase to at least 50% the levels obtained following a mixed infection with AdMA23 and AdCre1.

Because of the greater sensitivity and range of luciferase versus β-galactosidase assays, more detailed characterization of the lines was carried out using vector, AdMA19, that expresses luciferase following Cre mediated excision of a "spacer" DNA sequence. Parental 293 and 293N3S cell lines served as negative controls for levels of luciferase expressed from AdMA19 in the absence of Cre expression, and coinfections with AdMA19 and AdCrel were used as positive controls since it had been demonstrated previously that such mixed infections resulted in efficient excision of the spacer between the HCMV promoter and luciferase cDNA and induced high levels of luciferase expression.

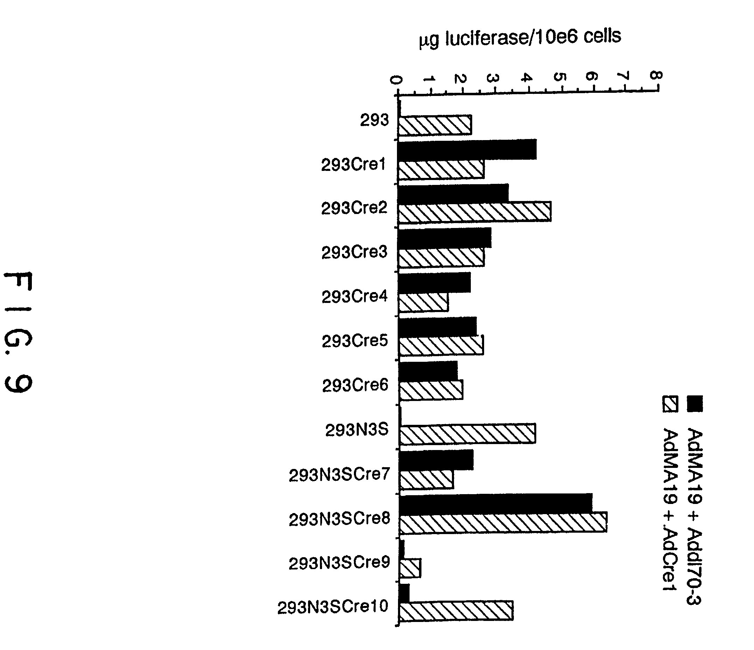

As in the β-galactosidase assays, Add170-3 was substituted for AdCre1 in functional assays for endogenous expression of Cre in transformed lines to maintain the total amount of infecting Ad5 at constant levels. The results of a typical analysis are shown in Fig. 9 for parental cells and the 10 transformed lines that had scored positive in the preliminary screening. After infection with AdMA19 and Add170-3 most of the transformed lines expressed luciferase at efficiencies comparable to levels seen in parental 293 or 293N3S cells doubly infected with AdMA19 and AdCre1 whereas mock infected lines, or parental lines doubly infected AdMA19 and Add170-3 did not express luciferase above background levels. Furthermore, infection of lines 293Cre1-6 and 293N3SCre7-8 with AdMA19 and AdCre1 did not significantly increase the amounts of luciferase made over those induced by infection with AdMA19 and Add170-3, in agreement with the results of the Western blot analysis showing levels of endogenously expressed Cre in lines 108 were similar to levels produced in AdCre1 infected 293 cells. Lines 293N3SCre9 and 10, on the other hand, initially expressed only low levels of luciferase following infection with AdMA19 and Add170-3, and significantly higher levels (5 fold and 12 fold greater, respectively) when Add170-3 was substituted with AdCre1 (Fig. 9).

Presently, these lines are no longer capable of inducing Cre responsive vectors without AdCre1 infection. This is consistent with current Western blot analysis (Fig. 8, lanes 11-12), which indicated a lack of Cre expression in these lines. It is likely that the lines 293N3SCre9 and 293N3SCre10 initially contained functioning Cre expression cassettes that were lost on continued passaging of the cells.

### Example 2. Stability of Cre Cell Lines.

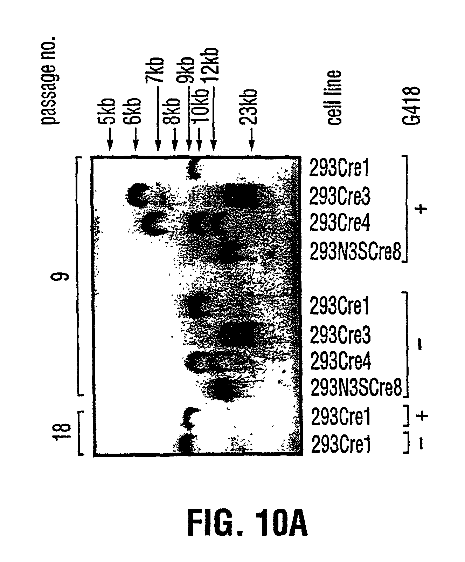

The stability of the Cre cell lines after multiple passages in media with or without G418 was determined. DNA from 293Cre1, 293Cre3, and 293Cre4 and 293N3SCre8 was isolated after 9 or more passages with or without G418 selection. For digestion, EcoR1, which does not cut within pLC3 was chosen in order to detect changes in the copy number of the *cre* gene easily. A digoxigenin-dUTP labelled probe was made using the 1.6 kb XbaI/SalI fragment of pSP13cre, and hybridized to EcoR1 restricted chromosomal DNA. The probe hybridized with an approximately 9.5 kb fragment of 293Cre1, and to a 15 kb fragment of 293N3SCre8, indicating that *cre* sequences were present in genomic DNA of these lines (Fig. 10A). The probe also detected a very faint band at approximately 18 kb in 293Cre1, which may represent either an insert of a subfragment of the *cre* gene or incompletely digested DNA. Southern blot analysis of HindIII restricted DNA, which cuts once in pLC3 at a site outside *cre* and flanking regulatory sequences, indicated that *cre* sequences were present at a single copy in 293Cre1, and approximately 3 copies in 293NSCre8. Both the 9.5 kb EcoRI fragment of 293Cre1 and the 15 kb fragment of 293N3SCre8 remained unchanged in cells maintained in the absence of G418 for 9 passages, with DNA for 293Cre1 retaining *cre* sequences after 18 passages. The *cre* probe detected intense bands at over 23 kb and 5 kb in 293Cre3, and intense 12.5, 9.8, and 6.6 kb bands from DNA of 293Cre4 cells maintained under G318 selection, indicating that *cre* sequences were present in these lines at 2 and 3 copies respectively (Fig. 10A). These results were confirmed by hybridization of the probe to HindIII restricted DNA. The fainter bands detected at 20, 18, and 15 kb in 293Cre3 cells may represent partially restricted DNA. When cells were passaged 9 times in the absence of G418, the 6 kb fragment of 293Cre3 and the 6.6 kb fragment of 293Cre4 were no longer detected, indicating that the corresponding *cre* inserts in these cell lines had been lost. The remaining *cre* specific fragments within 293Cre3 and 294Cre4 appeared to be stable. Assays of plating efficiencies of lines 293Cre 1, 3, and 4, and 293N3SCre8 all indicated that G418 resistance was a stable phenotype for all lines, regardless of whether the cells were maintained in selective or nonselective media.

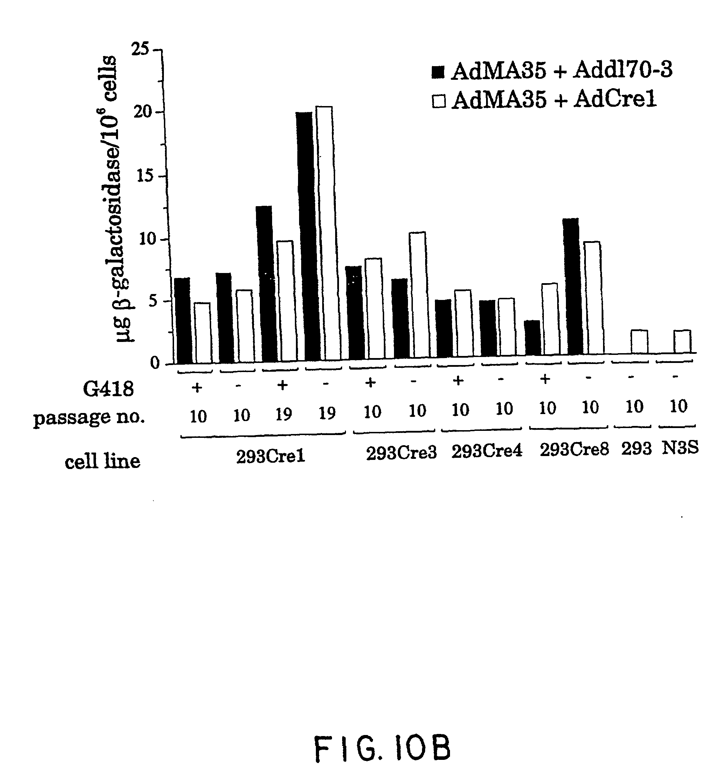

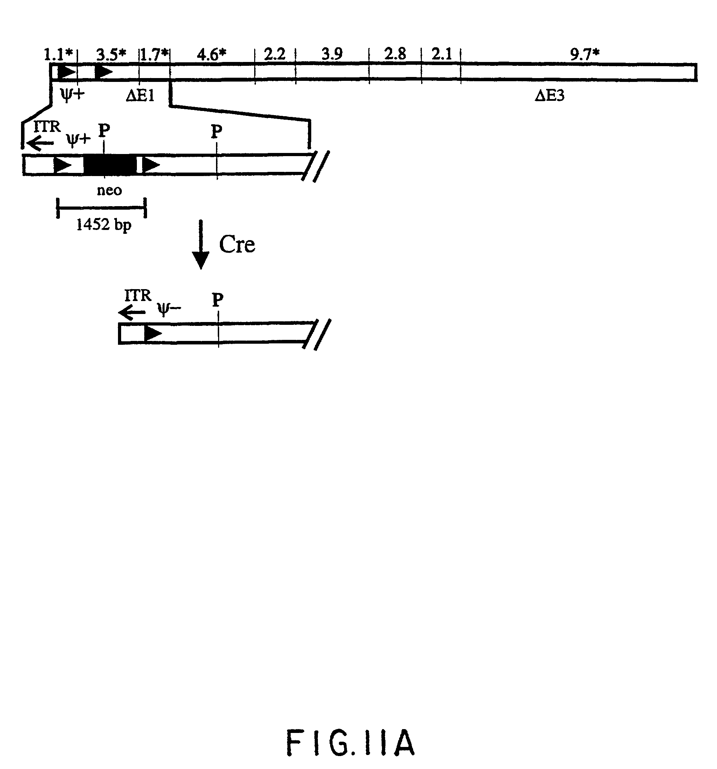

Several 293Cre lines grown with or without G418 selection were also tested for functional Cre activity. Cells were infected with a new β-galactosidase expression vector, AdMA35, in combination with Add170-3 or AdCre1, at an MOI of 10 pfu per cell for each virus as described previously. Cells were harvested 24 hours post-infection and β-galactosidase levels were determined. The results shown in Fig. 10B indicate that ability to induce expression of β-galactosidase from AdMA35 was generally unaltered after 10 or more passages in the absence of G418 selection. Functional activity of Cre in lines 293Cre1 and 293N3SCre8 was consistent with the stable presence of *cre* in Southern blot analysis. Thus *cre* sequences are stably integrated in these lines. The loss of one of two *cre* specific inserts in line 293Cre3 as detected by Southern blot (Fig. 10A), may coincide with a slight decrease in Cre function of these cells, as determined by assays with Cre inducible reporter genes (Fig. 10B) whereas the loss of one *cre* specific band in 293Cre4 appeared to have had no effect upon levels of functional Cre. It is possible that sufficient Cre is expressed from the stable inserts to bind all *loxP* sites introduced into this cell line. Alternatively, the unstable insert may not express any functional recombinase, and the level of Cre expression in 293Cre4 cells may be unchanged.

### Example 3. Construction of AdLC8 and AdLC8c Helper Viruses.

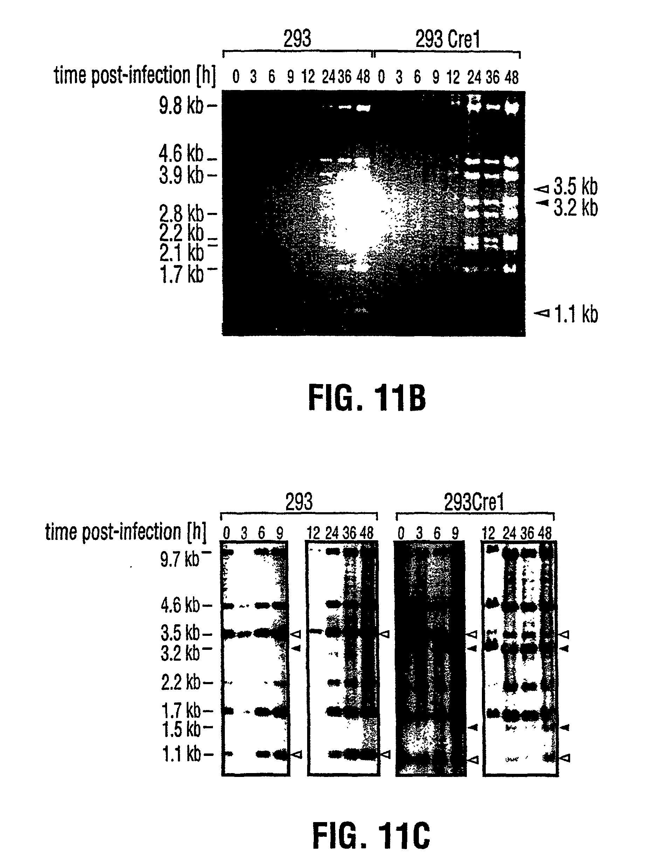

The plasmids were constructed according to standard protocols (Sambrook et al. 1989). The AdLC8 (Fig. 11A) helper virus was rescued by cotransfection of 293 cells with pLC8 having a "floxed" packaging signal (Ψ), and pBHG10. pLC8 was constructed as follows. A 9.6 kb AscI fragment was removed from pBG18 (Bett and Graham unpublished) a plasmid containing the majority of the adenovirus genome, including the Ad packaging signal and ITRs, generating pLC4. A synthetic *lox*P site with BamHI compatible ends was obtained by annealing two single-stranded oligonucleotides: 5'-GAT CCA ATA ACT TCG TAT AGC ATA CAT TAT ACG AAG TTA TAA GTA CTG AAT TCG-3' and 5'-GAT CCG AAT TCA GTA CTT ATA ACT TCG TAT AAT GTA TGC TAT ACG AAG TTA TTG-3'. The *loxP* site was cloned into the unique BamHI site of pLC4, located at nt 188 from the left end of the Ad5 genome, generating pLC5. This region has been shown previously to tolerate similar insertions of DNA without affecting virus replication (Bautista et al 1991). A second *lox*P site was inserted into pLC5 by first introducing the *lox*P oligonucleotides into the BamHI site of pABS.9, generating pLC7, and subcloning a 1.4 kb XbzI fragment, containing the loxP site and a neo bacterial-expression cassette, into the unique XbaI site of pLC5. Thus, the resulting plasmid, pLC8, contains two *lox*P sites separated by 1452 bp and flanking the adenovirus packaging signal and neo cassette. The AdLC8luc helper virus is similar to AdLC8, but lacks the neo expresion cassette in E1, and contains the firefly luciferase gene under the regulation of the human cytomegalovirus (HCMV) major immediate-early promoter (-299 to +72 bp relative to the transcription start site) and simian virus 40 polyadenylation signal (pA) inserted into the E3-deleted region. AdLC8cluc was rescued by contransfection of 293 cells with pLC8c and pUMA7L. pLC8c is a derivative of pLC8 from which the neo cassette was removed by digestion with Swal followed by recircularization. pUMA7L is a derivative of pBHG10 that contains firefly luciferase gene (DeWet et al. 1987) under the regulation of the HCMV immediate-early promoter and SV40 pA. The 2.2 kb BglII fragment, containing the HCMV-luciferase expression cassette was removed from pCA18 (Addison and Graham, unpublished) and ligated into the unique BamHI site of pABS.10 (Bett 1995), generating pABS101ucr. pABS.101ucr was then partially digested with PvuI in order to linearize the plasmid, and inserted into the PacI site of pBHG10, generating pUMA7L.

### Example 4. Construction of a Helper-Dependent Vector.

pCA35, which contains the *E. coli β*-galactosidase (lacZ) open reading frame under the control of the murine cytomegalovirus (MCMV) immediate-early promoter, was digested with SalI, repaired with the Klenow fragment of DNA polymerase, and recircularized, generating pCA35KS. The MCMV-lacZ expression cassette was excised from pCA35KS by digestion with XbaI/BamHI and inserted into XbaI/BamHI digested pABS.4, generating pABS.4MClacZ. This plasmid was then linearized with SalI and ligated into the unique XhoI site of pFG140dx3, a derivative of pFG140 (Graham 1984) that is deleted for sequences between 16.1 and 83.6 map units of the Ad5 genome, generating pUMA10R. A 1276 kb SwaI fragment from pUMA10R was removed and replaced by a 8270 bp SmaI fragment from bacteriophage lambda DNA (31617 to 39888 bp of the conventional lambda map), generating pRP1001.

### Example 5. Efficiency of Excision of Packaging Signals from AdLC8.

The kinetics and efficiency of excision by the Cre recombinase in the 293Crel cell line, and the ability of Ψ viral DNA to replicate. 293Cre1 cells, and 293 cells as controls, were infected with AdLC8 at an MOI of 10 PFU per cell. DNA was purified at various times post-infection, digested with PvuI, separated on a 0.8% agarose gel and the products examined by ethidium bromide staining. Fig. 11B demonstrates that excision of the *lox*P flanked segment is almost complete as evidenced by complete elimination in 293 Cre1 cells of the 1.1 kb band and almost complete disappearance of the 3.5 kb band both constituting the left end of the parental virus AdLC8. Instead, a 3.2 kb band is detectable from 12 hr onwards, which represents the viral band generated by excision of the floxed cassette. The excision product of 1.5 kb is not detectable on the agarose gel since it remains unreplicated due to a lack of an origin for DNA synthesis. Furthermore, Fig. 11B shows that the recombined virus (AdLC8Ψ) in the 293Cre1 line, replicates as well as the parental AdLC8 virus on 293 cells.

Southern blot analysis was performed to further examine the kinetics of Cre-mediated recombination. The agarose gel shown in Fig. 11B was transferred to a nylon membrane and probed with a partially-purified 4.4 kb BstXI-BglII labelled fragment from pLC8. The probe hybridized with fragments of 3489 and 1123 bp of AdLC8, and with a 1452 bp (representing the excised circle) and a 3160 bp fragment of the recombined virus (Fig. 11C). Furthermore, this probe hybridized with a 9777 bp fragment representing the right end of both viruses. Additional bands of approximately 4.6, 2.2, and 1.7 kb were detected due to contamination of the probe with the 5.3 kb BstXI fragment of pLC8. Over-exposure of membrane during autoradiography revealed that no recombination products were detectable prior to 6 hr post-infection in the 293Cre1 line. This suggest that the extreme left end of the virus may not be accessible by Cre during early times after infection, even though Cre is produced constitutively by 293Cre1 cell line and is present at the time of infection. Analysis of later time point using a shorter exposure time demonstrated that by 12 hr post-infection approximately 80% of the viral DNA had undergone recombination, and this fraction did not increase at later time points. The excised circle represented by a 1.5 kb band is detectable from 9 hr onward by the more sensitive Southern blot technique, and does not appear to be replicated. No recombination products (3.2 kb and 1.5 kb bands) are detectable after infection of 293 cells with AdLC8, indicating that the recombination products observed in the infected 293Cre1 cells derive from Cre-specific events. Since the quantity of unrecombined viral DNA increases with time with kinetics similar to that of the total DNA, we conclude that this represents virus that has entered the cell and is replicated, and is not due to residual virus that has not infected the cell. It is not known at present why these viral genomes escape Cre-mediated recombination, but it may reflect saturation of the Cre protein in the 293 cell line. Nevertheless, this illustrates that the packaging signal is efficiently excised from the majority of the ADLC8 virus in the 293Cre1 cell line, and removal of this sequence does not affect viral DNA replication.

### Example 6. Amplification of AdRP1001 by AdLC8 and AdLC8cluc.

Semiconfluent monolayers of 293 cells (60 mm) were transfected by calcium phosphate precipitation (Graham and van der Eb 1973) with 5µg of pRP1001 for 4 hr at 37°C, the transfected cells were infected at a multiplicity of infection (MOI) of 10 with AdLC8 or AdLC8cluc, the medium replaced, and the cells incubated until the monolayers showed complete CPE (48-72 hours). The cells were scraped into the medium and the virus released by 3 rounds of freezing and thawing. An aliquot of the resulting crude viral lysate (500 µl) was serially passaged on 60 mm dishes of 293Cre1 or 293Cre4 cells. During each round of amplification of the helper-dependent vector, the 293Cre cells were coinfected with AdLC8 or ADLC8cluc at an MOI of 5 for the first 2 rounds of amplification and at MOI of 1 for subsequent passages. Amplification of AdRP1001 was monitored by assaying an aliquot of the crude viral lysate after each passage for the presence of lacZ-expressing virus (blue forming units, BFU) on 293 cells. 293 monolayers were infected for 30 minutes with the virus inoculum (500µl), and the infected monolayers incubated for 24 hours at 37°C. The infected monolayers were washed once the PBS²⁺, fixed with 0.5 ml of 0.2% glutaraldehyde, 2% para-formaldehyde, 2mM MgCl₂ for 5 minutes at 37°C, washed, and stained with X-gal (5mM K₄Fe(CN)₆, 5mM K₃Fe(CN)₆, 2mM MgCl₂, 1 mg/ml 5-bromo-4-chloro-3-indolyl-β-D-galactopyranoside [X-gal] in PBS). Plates were incubated overnight at room temperature, and the number of BFU determined by visual inspection. Viral lysates were also monitored for the presence of helper virus and RCA by plaque assays on 293 lysates were also monitored for the presence of helper virus and RCA by plaque assays on 293 and A549 monolayers, respectively. The frequency of AdLC8-mediated conversion of PRP1001 into packagable linear genomes was about 800 BFU/µg transfected DNA.

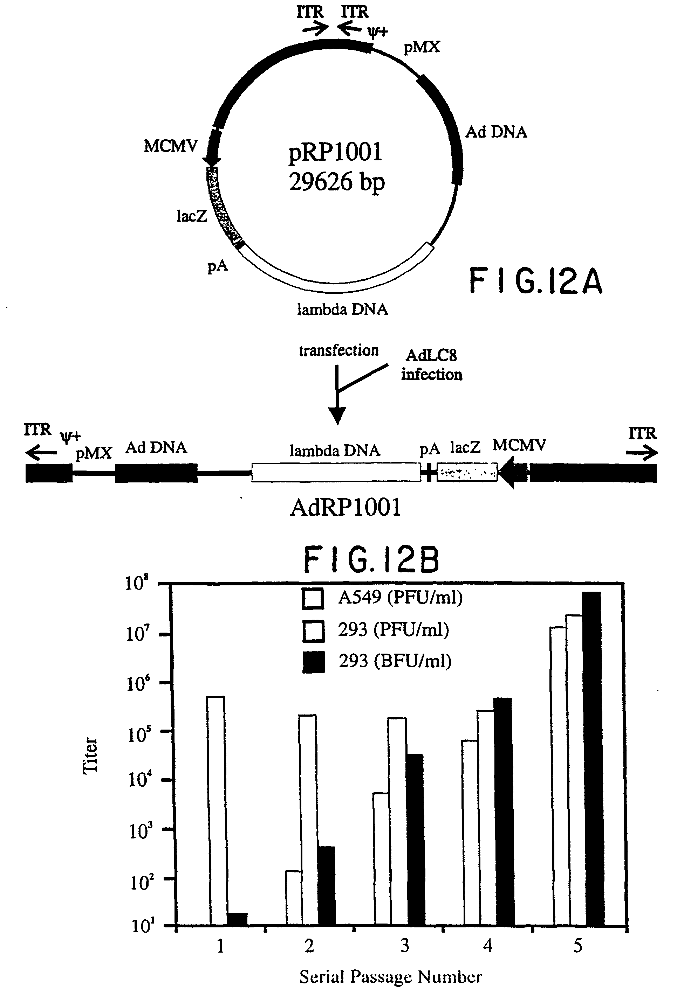

293Cre1 cells were transfected with pRP1001, a plasmid that is deleted for most of the adenovirus sequences (Fig. 12A) and then infecting the transfected cells with the AdLC8 helper virus 18 hours later. Serial passage of AdRP1001 through AdLC8-infected 293Cre1 cells resulted in an increase in the titer of helper-dependent vector at each passage (Fig. 12B). After five serial passages, the titer of AdRP1001 was approximately 10⁸ BFU/ml. The quantity of AdLC8 that had escaped the excision of packaging signals and the formation of RCA was monitored by plaque assays on 293 and A549 cell lines respectively. The level of AdLC8 present after each passage remained relatively constant at 5 x 10⁵ PFU/ml (Fig. 12B), and was comparable to the relative amount of unrecombined AdLC8 DNA observed during Southern blot analysis (Fig. 11C). RCA were detected after the second amplification through 293Cre1, and RCA titers continued to increase with subsequent passages. That this was indeed RCA was determined by analysis of the DNA content of several plaque isolates. Restriction enzyme analysis of DNA from cesium chloride-purified virus also showed that the final viral preparation contained restriction patterns consistent with the presence of 3 distinct viral populations: AdRP1001, AdLC8, and E3-deleted RCA.

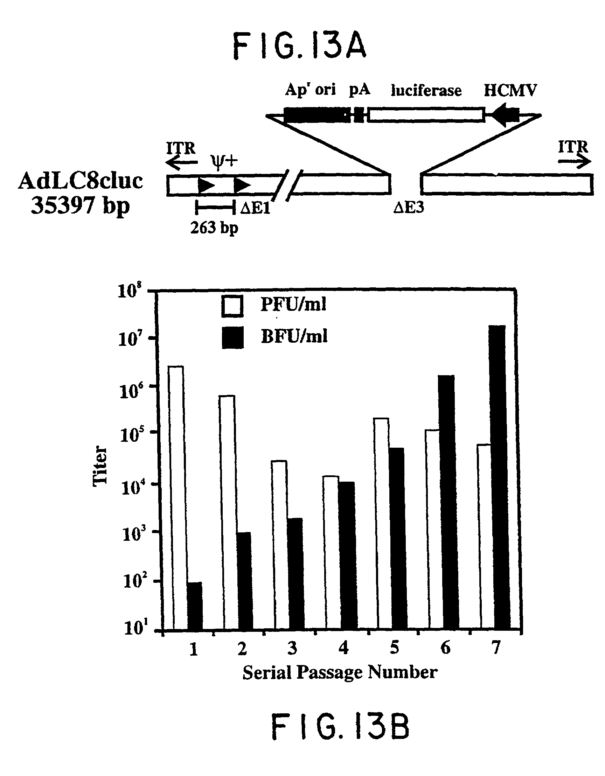

Using the same amplification strategy outlined for AdLC8, the AdRP1001 vector was amplified to high titers with AdLC8cluc. (Fig. 13A) The frequency of AdLC8cluc-mediated conversion of pRP1001 into packagable linear genomes was similar to that of AdLC8 (about 1400 BFU/µg of transfected DNA in AdLC8cluc-infected 293 cells). Serial passage of AdRP1001 through AdLC8cluc-infected 234Cre1 cells resulted in an approximately 10-fold increase in the titer of AdRP1001 per passage. Similar kinetics of amplification were observed for other vectors ranging in size from 27.7 kb to 33.6 kb. Furthermore, the quantity of AdLC8cluc present in stocks remained low, and during the seventh passage, constituted less than 1% of the total virus. Finally, no RCA were detected. The data presented in Fig. 13B clearly illustrates that AdLC8cluc may be employed as a helper virus to produce high titer stocks of AdRP1001.

### Example 7. Large Scale Virus Preparations of AdRP1001.

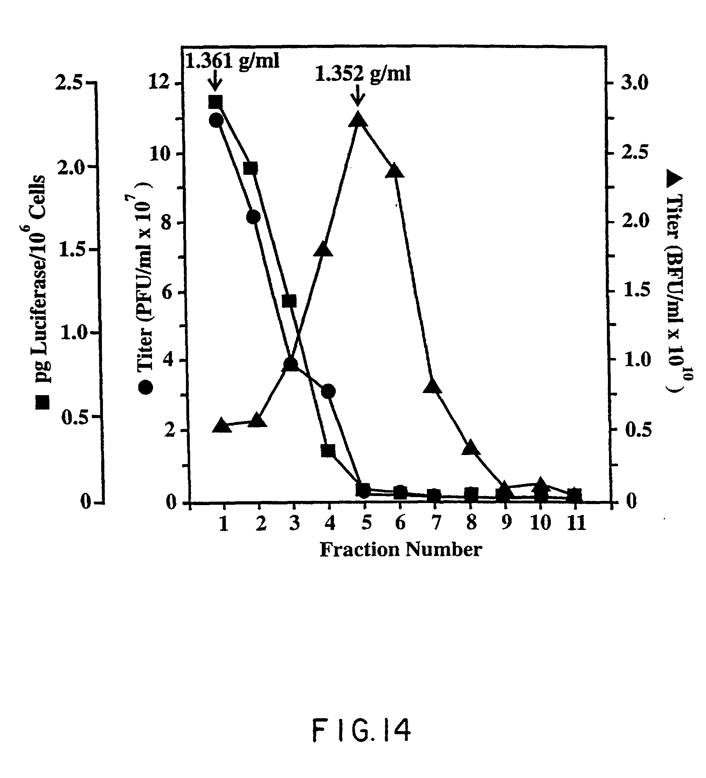

Large scale virus preparations of AdRP1001 were performed by infecting 150 mm dishes of 293Cre with 1 ml of crude AdRP1001 stock per 150 mm dish (MOI = ∼1) supplemented with 2 x 10⁷ PFU of helper virus. After complete CPE, purification of AdRP1001 virions was performed by CsCl buoyant density centrifugation as previously described. After centrifugation, fractions were collected through the viral bands, and assayed for the presence of luciferase-expressing virus. Each fraction was diluted 1:1000, and an aliquot (500µl) was used to infect 293 monolayers. Twenty-four hours post-infection, crude protein extracts were prepared from the infected cells and assayed for luciferase activity using a commercial kit (Promega) and a Berthold model LB9501 luminometer. Luciferase activity (R² = 0.963) correlated well with the relative titer of AdlC8cluc, indicating that luciferase activity is a good indicator of the level of contaminating helper virus. Using the relationship shown in Fig. 14, 1 PFU AdLC8cluc = ∼2.6 x 10⁵ pg luciferase per 10⁶ infected cells.

AdRP1001 and helper virus titers were also isolated from the lower density band after CsCl centrifugation. We obtained a titer of 8.6 x 10⁹ BFU/ml, or a total yield of 6.5 x 10⁹ AdRP1001 virions from 20-150 mm dishes of 293Cre cells. The ratio of AdRP1001 to AdLC8cluc in this stock was ∼1:16,000, or a 30-fold enhancement in the purity of AdRP1001 over the crude stock used in the purification. This is in contrast to helper-dependent systems developed previously in which the major component of the "purified" vector was helper virus, with between 100 to 1000 fold more helper virus than vector. (Mitani et al. 1995, Fisher et al. 1996). In addition, our yield of nearly 10¹⁰ virions is ∼1000 fold higher than that obtained using these other systems. Furthermore, it has been shown that the vectors in these systems underwent DNA recombination at high frequencey, generating a heterogeneous population of vector consisting of monomer, dimer and more complex DNA structures. In contrast, restriction analysis of purified AdRP1001 showed that the DNA structure of the vector was identical to passage vector and restriction maps predicted from the sequence of the initial transfecting plasmid pRP1001. (Fig. 4B) We conclude that the helper-dependent system described above is capable of producing high titer vector preparations that contain very low quantities of helper virus.

## Claims

1. A recombinant adenovirus vector system for expressing foreign genes, comprising:
(a) a first adenovirus vector having a modified early region 1 (E1) in which the packaging signals of the virus are flanked on either side by target sites that are recognized by a recombinase, and
(b) a cell line that is able to support replication of the virus of 1 (a) and which additionally expresses the said recombinase that is able to catalyze site specific recombination between its target sites, and
(c) a second vector, having
(i) a deletion of large portions of the viral genome, up to approximately 35,000 bp but retaining Ad viral sequences required in cis for viral DNA replication and packaging of viral nucleic acid into virions, namely the left and right ITRs and the packaging signal and
(ii) a fragment or fragments of foreign nucleic acid of about 35,000 bp in size.

2. A recombinant adenovirus vector system according to claim 1, wherein the recombinase is Cre and the target sites are *loxP.*

3. A recombinant adenovirus vector system according to claim 1 or claim 2 wherein the second vector of part 1 (c) is derived from a plasmid comprising the left and right ITRs and packaging signal of the viral genome, a plasmid origin of replication and an antibiotic resistance gene, and a foreign nucleic acid of up to 35,000 bp in length.

4. A plasmid used for making a vector (helper virus) according to claim 1 (a), said plasmid comprising a modified circular adenovirus genome wherein sequences from region E3 of the viral genome are replaced by (a) the packaging signal from the viral genome bracketed by target sites for a recombinase, (b) a bacterial plasmid origin of replication and (c) an antibiotic resistance gene.

5. A plasmid according to claim 4, wherein the recombinase is Cre, the target sites are *loxP* and the antibiotic is ampicillin.

6. Use of a plasmid comprising the left and right ITRs and packaging signal of an adenoviral genome, a plasmid origin of replication and an antibiotic resistance gene, and a foreign nucleic acid of up to 35,000 bp in length in the manufacture of a vector system as claimed in claim 1 or claim 2.

7. A method of producing a helper virus genome that is incapable of packaging its nucleic acid into virions but retains viral functions that can act in trans to complement the replication of a second, helper dependent vector from which all or most of the viral genes have been deleted and substituted with a foreign nucleic acid, comprising:
co-infecting a cell line that supports replication of an adenovirus vector with (a) a first vector having a modified early region 1 (E1) such that the packaging signals of the virus are flanked on either side by target sites that are recognized by a recombinase, and (b) a second vector, having
(i) a deletion of large portions of the viral genome, up to approximately 35,000 bp but retaining Ad viral sequences required in cis for viral DNA replication and packaging of viral nucleic acid into virions, namely the left and right ITRs and the packaging signal, and
such that the target sites flanking the packaging signal of the first vector will be acted upon by a recombinase expressed in said cells to induce excision of said packaging signal.

8. A method according to claim 7, wherein the recombinase is Cre and the target sites are *loxP*

## Patentansprüche

1. Rekombinantes Adenovirusvektorsystem zur Expression von Fremdgenen Gene, umfassend:
(a) einen ersten Adenovirusvektor mit einer modifizierten frühen Region 1 (E1), worin die Verpackungssignale des Virus auf jeder Seite durch Zielstellen flankiert sind, die durch eine Rekombinase erkannt werden, und
(b) eine Zelllinie, die die Replikation des Virus von 1(a) unterstützen kann und die zusätzlich die Rekombinase exprimiert, die eine ortsspezifische Rekombination zwischen Ihren Zielstellen katalysieren kann, und
(c) einen zweiten Vektor mit
(i) einer Deletion großer Teile des Virusgenoms bis zu ungefähr 35000 Bp, jedoch unter Erhaltung von Ad-Virussequenzen, die in cis erforderlich sind für die Virus-DNA-Replikation und das Verpacken der Virusnukleinsäure in Virionen, nämlich die linken und rechten ITR und das Verpackungssignal, und
(ii) einem Fragment oder Fragmenten einer Fremdnukleinsäure mit einer Größe von etwa 35000 Bp.

2. Rekombinantes Adenovirusvektorsystem nach Anspruch 1, worin die Rekombinase Cre ist und die Zielstelle loxP sind.

3. Rekombinantes Adenovirusvektorsystem nach Anspruch 1 oder Anspruch 2, worin der zweite Vektor von Teil 1(c) aus einem Plasmid stammt, umfassend die linken und rechten ITR und das Verpackungssignal des Virusgenoms, einen Plasmidreplikationsursprung und ein Antibiotikaresistenzgen und eine Fremdnucleinsäure mit einer Länge von bis zu 35000 Basenpaaren.

4. Plasmid, das verwendet wird zur Herstellung eines Vektors (Helfervirus) nach Anspruch 1(a), wobei das Plasmid ein modifiziertes zirkuläres Adenovirusgenom umfasst, worin Sequenzen der Region E3 des Virusgenoms ersetzt sind durch (a) das Verpackungssignal des Virusgenoms, das durch die Zielstellen für eine Rekombinase eingefasst ist, (b) einen Bakterienplasmidreplikationsursprung und (c) ein Antibiotikaresistenzgen.

5. Plasmid nach Anspruch 4, worin die Rekombinase Cre ist, die Zielstellen loxP sind und das Antibiotikum Ampicillin ist.

6. Verwendung eines Plasmids, umfassend die linken und rechten ITR und das Verpackungssignal eines Adenovirusgenoms, einen Plasmidreplikationsursprung und ein Antibiotikaresistenzgen und eine Fremdnucleinsäure mit einer Länge von bis zu 35000 Bp bei der Herstellung eines Vektorsystems nach Anspruch 1 oder 2.

7. Verfahren zur Herstellung eines Helfervirusgenoms, das nicht in der Lage ist zur Verpackung seiner Nukleinsäure in Virionen jedoch seine viralen Funktionen beibehält, das in trans wirken kann, um die Replikation eines zweiten, helferabhängigen Vektors zu ergänzen, von welchem alle oder die meisten Virusgene entfernt oder substituiert worden sind mit einer Fremdnucleinsäure, umfassend:
Coinfizieren einer Zelllinie, die die Replikation eines Adenovirusvektors unterstützt mit
(a) einem ersten Adenovirusvektor mit einer modifizierten frühen Region 1 (E1), worin die Verpackungssignale des Virus auf jeder Seite durch Zielstellen flankiert sind, die durch eine Rekombinase erkannt werden, und
(b) einem zweiten Vektor mit
(i) einer Deletion großer Teile des Virusgenoms bis zu ungefähr 35000 Bp, jedoch unter Erhaltung von Ad-Virussequenzen, die in cis erforderlich sind für die Virus-DNA-Replikation und das Verpacken der Virusnukleinsäure in Virionen, nämlich die linken und rechten ITR und das Verpackungssignal und
derart, dass auf die Zielstellen, die das Verpackungssignal des ersten Vektors flankieren, durch eine Rekombinase eingewirkt wird, die in den Zellen expimiert wird, um ein Herausschneiden des Verpackungssignals zu induzieren.

8. Verfahren nach Anspruch 7, worin die Rekombinase Cre ist und die Zielstellen loxP sind.

## Revendications

1. Système de vecteur adénoviral recombinant pour l'expression de gènes étrangers, comprenant :
(a) un premier vecteur adénoviral ayant une région précoce 1 modifiée (E1) dans laquelle les signaux d'encapsidation du virus sont entourés de chaque côté par des sites cibles qui sont reconnus par une recombinase, et
(b) une lignée cellulaire qui est capable d'assurer la réplication du virus de 1 (a) et qui exprime en outre ladite recombinase qui est capable de catalyser la recombinaison spécifique de sites entre ses sites cibles, et
(c) un second vecteur, ayant
(i) une délétion de parties importantes du génome viral, jusqu'à approximativement 35 000 pb mais conservant les séquences virales Ad requises en cis pour la réplication de l'ADN viral et l'encapsidation de l'acide nucléique viral dans des virions, à savoir les ITR gauche et droit et le signal d'encapsidation, et
(ii) un ou plusieurs fragments d'acide nucléique étranger ayant des dimensions d'environ 35 000 pb.

2. Système de vecteur adénoviral recombinant suivant la revendication 1, dans lequel la recombinase est la recombinase Cre et les sites cibles sont des sites loxP.

3. Système de vecteur adénoviral recombinant suivant la revendication 1 ou la revendication 2, dans lequel le second vecteur de la partie 1 (c) est dérivé d'un plasmide comprenant les ITR gauche et droit et le signal d'encapsidation du génome viral, une origine plasmidique de réplication et un gène de résistance aux antibiotiques, et un acide nucléique étranger ayant une longueur allant jusqu'à 35 000 pb.

4. Plasmide utilisé pour la préparation d'un vecteur (virus auxiliaire) suivant la revendication 1 (a), ledit plasmide comprenant un génome d'adénovirus circulaire modifié dans lequel les séquences de la région E3 du génome viral sont remplacées par (a) le signal d'encapsidation du génome viral entouré par des sites cibles pour une recombinase, (b) une origine plasmidique bactérienne de réplication et (c) un gène de résistance à un antibiotique.

5. Plasmide suivant la revendication 4, dans lequel la recombinase est la recombinase Cre, les sites cibles sont les sites loxP et l'antibiotique est l'ampicilline.

6. Utilisation d'un plasmide comprenant les ITR gauche et droit et le signal d'encapsidation d'un génome adénoviral, une origine plasmidique de réplication et un gène de résistance à un antibiotique, et un acide nucléique étranger ayant une longueur allant jusqu'à 35 000 pb dans la production d'un système de vecteur suivant la revendication 1 ou la revendication 2.

7. Procédé pour la production d'un génome de virus auxiliaire qui est incapable d'encapsider son acide nucléique dans des virions mais qui conserve des fonctions virales pouvant agir en trans pour la complémentation de la réplication d'un second vecteur, sous la dépendance de l'auxiliaire, duquel la totalité ou la plus grande partie des gènes viraux ont été éliminés par délétion et remplacés par un acide nucléique étranger, comprenant :
la co-infection d'une lignée cellulaire qui assure la réplication d'un vecteur adénoviral avec (a) un premier vecteur ayant une région précoce 1 modifiée (E1) de telle sorte que les signaux d'encapsidation du virus soient entourés de chaque côté par des sites cibles qui sont reconnus par une recombinase, et (b) un second vecteur, ayant
(i) une délétion de parties importantes du génome viral, jusqu'à approximativement 35 000 pb mais conservant les séquences virales Ad requises en cis pour la réplication de l'ADN viral et l'encapsidation de l'acide nucléique viral dans des virions, à savoir les ITR gauche et droit et le signal d'encapsidation, et
de telle sorte que les sites cibles entourant le signal d'encapsidation du premier vecteur soient sollicités par une recombinase exprimée dans lesdites cellules pour induire l'excision dudit signal d'encapsidation.

8. Procédé suivant la revendication 7, dans lequel la recombinase est la recombinase Cre et les sites cibles sont les sites loxP.
